(19) Europäisches Patentamt / European Patent Office / Office européen des brevets

(11) EP 2 674 489 A1

(12) EUROPEAN PATENT APPLICATION

(43) Date of publication:
**18.12.2013 Bulletin 2013/51**

(51) Int Cl.:
***C12N 9/90*** *(2006.01)* ***C12N 15/63*** *(2006.01)*
***C12P 7/42*** *(2006.01)* ***C12P 7/52*** *(2006.01)*

(21) Application number: **12173010.5**

(22) Date of filing: **21.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.06.2012 EP 12172192**

(71) Applicant: **Evonik Industries AG**
**45128 Essen (DE)**

(72) Inventors:
- **Haas, Thomas, Dr.**
  **48161 Münster (DE)**
- **Schiemann, Yvonne, Dr.**
  **45357 Essen (DE)**
- **Przybylski, Denise**
  **04416 Merkkleeberg (DE)**
- **Rohwerder, Thore, Dr.**
  **04299 Leipzig (DE)**
- **Müller, Rolad, Prof.**
  **04425 Taucha (DE)**
- **Harms, Hauke, Prof.**
  **04105 Leipzig (DE)**

(54) **Biotechnological 2-hydroxyisobutyric acid production**

(57) The present invention relates to a knallgas bacterium or acetogenic bacterium expressing a 2-hydroxyisobutyryl-coenzyme A mutase, a method for the production of 2-hydroxyisobutyric acid, comprising contacting in an aqueous medium the knallgas bacterium or acetogenic with a gas mixture comprising hydrogen and carbon dioxide and the use of the knallgas bacterium or acetogenic bacterium for the production of 2-hydroxyisobutyric acid.

EP 2 674 489 A1

**Description**

The present invention relates to a knallgas bacterium or acetogenic bacterium expressing a 2-hydroxyisobutyryl-coenzyme A mutase, a method for the production of 2-hydroxyisobutyric acid, comprising contacting in an aqueous medium the knallgas bacterium or acetogenic with a gas mixture comprising hydrogen and carbon dioxide and the use of the knallgas bacterium or acetogenic bacterium for the production of 2-hydroxyisobutyric acid.

Growing global population and worldwide raising life standards inevitably enforce the conflict of satisfying the people's demands for goods and services on one and for sustainable development and considerate treatment of nature and earth's resources on the other hand. It is not only that fossil carbon sources will become limited in the future, but also the accessibility of nowadays treated and prospected sites will increasingly be hindered. Known accidents drastically demonstrate this danger such as the recent oil disaster in the Gulf of Mexico. White biotechnology offers an elegant way to provide alternatives not only due to the application of microorganisms or components of cells but also due to the application of the broad spectrum of new generation renewable substrates.

For ages the chemical industry has produced, for instance alcohols and organic acids, in bulk dimensions in biotechnological processes mainly to be used as chemicals, but above all as energy carriers. Of those, bio-ethanol is a recent integrant of modern fuels for motor vehicles and bio-butanol is contemplated. Based on the knowledge of bulk scale energy carrier production recent intentions envision the extension of platform chemicals for wider application. Special attempts are directed towards the synthesis of chemicals such as 1,3-propanediol, succinate, gluconic acid or citric acid. Likewise, 2-hydroxyisobutyric acid (2-HIB) fits well into this scheme as it is rising as platform chemical. In particular since 2-HIB can be used as precursor for methacrylic acid, a monomeric compound required for the synthesis of such prominent products like Plexiglas® and as an important ingredient for coating materials, paint and glues. Generally, traditional biotechnological processes, such as those for bio-ethanol production, are based on carbon sources of the so-called first generation, i.e. carbohydrates derived directly from plants like sugars or starch. It turns out, however, that such resources are limited and that it seems adequate to channel them into the production of foodstuffs rather than chemicals.

The state of the art teaches biotechnological routes towards the production of 2-HIB. For example, PCT/EP2009/055089 teaches cell which has been genetically modified so as to be capable of producing more 2-hydroxyisobutyric acid or more polyhydroxyalkanoates containing 2-hydroxyisobutyric acid monomer units than its wild type, **characterized in that** 2-hydroxyisobutyric acid or polyhydroxyalkanoates containing 2-hydroxyisobutyric acid monomer units are produced via acetoacetyl-coenzyme A as intermediate and 3-hydroxybutyryl-coenzyme A as precursor. However, the process for the production of 2-HIB contemplates the use of carbohydrate-containing carbohydrates or derivates thereof, for examples sodium gluconate, which is to be avoided from an environmental point of view. Furthermore, a significant proportion of any carbon dioxide consumed in the production of said carbohydrates is not converted to the sought-after product 2-HIB, but lost in various metabolic routes on the way towards 2-HIB.

Therefore, the problem underlying the present invention is to devise a process for producing 2-HIB which may be carried out in the absence of energy-rich organic compounds. Moreover, the problem underlying the present invention is to devise a biotechnological route towards the production of 2-HIB. Moreover, the problem underlying the present invention is to devise a biotechnological method for the production of 2-HIB that is superior compared to state of the art processes in that more carbon atoms in the 2-HIB obtained are derived from carbon dioxide molecules, i.e. the proportion of carbon atoms derived from carbon dioxide rather than from any other organic molecules, for example alcohols or carbohydrates, is higher.

The problem underlying the present invention is solved by the subject matter of the attached claims.

In a first aspect, the problem underlying the present invention is solved by a knallgas bacterium or acetogenic bacterium expressing a 2-hydroxyisobutyryl-coenzyme A mutase.

In a first embodiment of the first aspect, the bacterium is genetically modified such that it has, compared to the respective wild type strain, a reduced capacity to synthesise polyhydroxybutyrate.

In a second embodiment of the first aspect, which is also an embodiment of the first embodiment, the bacterium has a polyhydroxybutyrate synthase gene knockout.

In a third embodiment of the first aspect, which is also an embodiment of the first to second embodiments, the bacterium is incapable of synthesising polyhydroxybutyrate.

In a fourth embodiment of the first aspect, which is also an embodiment of the first to third embodiments, the bacterium is a knallgas bacterium selected from the group comprising *Methanobacterium, Acetobacterium, Desulfovibrio, Desulfomonas, Paracoccus, Achromobacter, Alcaligenes, Pseudomonas, Nocardia* and *Cupriavidus,* and is preferably a *Cupriavidus* strain, most preferably *Cupriavidus necator* H16, or an acetogenic bacterium selected from the group comprising *Clostridium, Moorella* and *Acetoanaerobium, Acetobacterium, Archaeoglobus, Butyribacterium, Carboxydibrachium, Carboxydocella, Carboxydothermus, Citrobacter, Desulfotomaculum, Eubacterium, Methanosarcina, Methanothermobacter, Oxobacter Peptostreptococcus, Rhodopseudomonas, Rhodospirillum, Rubrivivax, Thermincola, Thermococcus, Thermolithobacter, Thermoanaerobacter and Thermosyntrophicum.*

In a 5th embodiment of the first aspect, which is also an embodiment of the first to fourth embodiments, the bacterium expresses the nucleic acid comprising SEQ ID NO 1 or a variant thereof or a polypeptide encoded by said nucleic acid or a variant of said polypeptide and expresses a nucleic acid comprising SEQ ID NO 2 or a variant thereof or a polypeptide encoded by the latter nucleic acid or a variant of the latter polypeptide.

In a 6th embodiment of the first aspect, which is also an embodiment of the first to 5th embodiments, the bacterium expresses, in addition to a 2-hydroxyisobutyryl-coenzyme A mutase, a MeaB protein, the polypeptide encoded by SEQ ID NO 3 or a variant thereof.

In a 7th embodiment of the first aspect, which is also an embodiment of the first to 6th embodiments,the bacterium expresses, the bacterium is capable of synthesizing 3-Hydroxybutyryl-Coenzym A.

In a 8th embodiment of the first aspect, which is also an embodiment of the first to 7th embodiments, the bacterium expresses, the bacterium expresses a β-ketothiolase and a acetoacetyl-CoA reductase.

In a second aspect, the problem underlying the present invention is solved by a method for the production of 2-hydroxyisobutyric acid, comprising

a) contacting in an aqueous medium the knallgas bacterium or acetogenic bacterium according to any of claims 1 to 9 with a gas mixture comprising hydrogen and carbon dioxide.

In a first embodiment of the second aspect, the hydrogen partial pressure in the gas mixture is 0.1 to 100 bar, more preferably 0.2 to 10 bar, most preferably 0.5 to 4 bar, and the carbon dioxide partial pressure is 0.03 to 100 bar, more preferably 0,05 to 1 bar, most preferably 0.05 to 0.3 bar.

In a second embodiment of the second aspect, which is also an embodiment of the first embodiment, the bacterium tolerates the presence of oxygen and aqueous solution is aerobic.

In a third embodiment of the second aspect, which is also an embodiment of the first to second embodiments, the bacterium tolerates the presence of oxygen and the gas mixture comprises oxygen in addition to hydrogen and carbon dioxide, and the oxygen partial pressure in the gas mixture is preferably 0.03 to 10 bar, more preferably 0.04 to 1 bar, most preferably 0.04 to 0.5 bar.

In a fourth embodiment of the second aspect, which is also an embodiment of the first to third embodiments, the aqueous medium does not comprise carbohydrates or, preferably, any carbon source other than carbon dioxide.

In a 5th embodiment of the first aspect, which is also an embodiment of the first to fourth embodiments, step a) is carried out in the absence of a growth-limiting nutrient other than a carbon.

In a 6th embodiment of the first aspect, which is also an embodiment of the first to 5th embodiments, step a) is carried out at 20 to 37 °C, more preferably 25 to 35 °C, most preferably at 28 to 32 °C.

In a third aspect, the problem underlying the present invention is solved by the use of the knallgas bacterium or acetogenic bacterium according to any aspect or embodiment of the invention for the production of 2-hydroxyisobutyric acid.

The present invention is based on the surprising finding that a knallgas bacterium or acetogenic bacterium expressing a 2-hydroxyisobutyryl-coenzyme A mutase may be used to produce 2-HIB.

Moreover, the present invention is based on the surprising finding that the yield of such a process may be increased by using for the production of 2-HIB a knallgas bacterium or acetogenic bacterium expressing a 2-hydroxyisobutyryl-coenzyme A mutase that has, compared to the respective wild type strain, a reduced capacity to synthesise polyhydroxybutyrate.

Moreover, the present invention is based on the surprising finding that the yield of such a process may be increased by using for the production of 2-HIB a knallgas bacterium or acetogenic bacterium expressing a 2-hydroxyisobutyryl-coenzyme A mutase subjected to growth-limiting conditions other than the absence of a carbon source.

The present invention centers around the use of a knallgas bacterium or acetogenic bacterium expressing a 2-hydroxyisobutyryl-coenzyme A mutase. These bacteria have in common the capability of metabolising a mixture comprising hydrogen and carbon dioxide to build up organic compounds.

The present invention contemplates both the use of a wild type and genetically modified acetogenic bacterium or knallgas bacterium. In a preferred embodiment, the term "acetogenic bacterium", as used herein, refers to a bacterium capable of using the Wood-Ljungdahl pathway, i.e. the pathway converting carbon monoxide, carbon dioxide and hydrogen to acetate. In a preferred embodiment, the activity of at least one enzyme involved in the Wood-Ljungdahl pathway is increased relative to the wild type cell. In a preferred embodiment, the term "enzyme involved in the Wood-Ljungdahl pathway", as used herein, comprises any enzyme that binds to or, preferably accepts as a substrate, one of the substrates of said pathways, preferably carbon monoxide, carbon dioxide or hydrogen, or any of the intermediates formed within the pathway starting from any of these substrates as the substrates are converted to acetate or derivatives thereof. In another preferred embodiment, the term "enzyme involved in the Wood-Ljungdahl pathway", as used herein, refers to an enzyme from the group comprising CO dehydrogenase and acetyl-CoA synthetase. In a preferred embodiment, the acetogenic bacterium is selected from the group comprising *Clostridium, Moorella* and *Acetoanaerobium, Acetobacte-*

*rium, Archaeoglobus, Butyribacterium, Carboxydibrachium, Carboxydocella, Carboxydothermus, Citrobacter, Desulfotomaculum, Eubacterium, Methanosarcina, Methanothermobacter, Oxobacter Peptostreptococcus, Rhodopseudomonas, Rhodospirillum, Rubrivivax, Thermincola, Thermococcus, Thermolithobacter, Thermoanaerobacter and Thermosyntrophicum.* Examplary acetogenic bacteria comprise, but are not limited to *Acetoanaerobium notera, Acetobacterium woodii, Archaeoglobus fulgidus, Butyribacterium methylotrophicum, Butyribacterium methyltrophicum, Carboxydibrachium pacificus, Carboxydocella sporoproducens, Carboxydocella thermoautotrophica, Carboxydothermus hydrogenoformans, Citrobacter sp. Y19, Clostridium aceticum, Clostridium acetobutylicum, Clostridium autoethanogenum, Clostridium carboxidivorans, Clostridium lungdahlii, Desulfotomaculum carboxydivorans, Desulfotomaculum kuznetsovii, Desulfotomaculum thermobenzoicum subsp., Thermosyntrophicum, Eubacterium limosum, Methanosarcina acetivorans C2A, Methanosarcina barkeri, Methanothermobacter thermoautotrophicus, Moorella AMP, Moorella thermoacetica, Moorella thermoautotrophica, Oxobacter pfennigii, Peptostreptococcus productus, Peptostreptococcus productus, Rhodopseudomonas palustris P4, Rhodospirillum rubrum, Rubrivivax gelatinosus, Thermincola carboxydiphila, Thermincola ferriacetica, Thermococcus AM4, Thermolithobacter carboxydivorans* and *Thermoanaerobacter kivui.*

In another preferred embodiment, the acetogenic bacterium is a bacterium genetically modified to be acetogenic, whilst the corresponding wild type strain is not. In this case, the bacterium may be any organism amenable to such modification, for example *E. coli.*

In a preferred embodiment, the term "knallgas bacterium", as used herein, refers to any bacterium capable of oxidising hydrogen, using oxygen as a terminal electron acceptor, and of fixing carbon dioxide under aerobic conditions. In a preferred embodiment, the knallgas bacterium is selected from the group comprising *Methanobacterium, Acetobacterium, Desulfovibrio, Desulfomonas, Paracoccus, Achromobacter, Alcaligenes, Pseudomonas, Nocardia* and *Cupriavidus,* and is preferably a *Cupriavidus* strain, most preferably *Cupriavidus necator* H16. Examplary knallgas bacteria comprise, but are not limited to *Acidovorax facilis, Acidovorax sp., Alcaligenes eutropha, Alcaligenes sp., Bradyrhizobium japonicum, Bradyrhizobium sp., Cupriavidus necator DSM 531, Heliobacter sp., Hydrogenobacter sp., Hydrogenobacter thermophilus, Hydrogenomonas eutropha, Hydrogenomonas pantotropha, Hydrogenomonas sp., Hydrogenomonas facilis, Hydrogenophaga sp., Hydrogenovibrio marinus (strain MH-110), Hydrogenovibrio sp, .Oxyhydrogen microorganism, Pseudomonas hydrogenothermophila, Pseudomonas hydrogenovora, Pseudomonas sp., Ralstonia eutropha, Ralstonia sp., Rhizobium japonicum, Rhizobium sp., Rhodococcus opacus, Rhodococcus sp., Rhodopseudomonas acidophila, Rhodopseudomonas blastica, Rhodopseudomonas capsulata, Rhodopseudomonas palustris, Rhodopseudomonas sheroides, Rhodopseudomonas sulfoviridis, Rhodopseudomonas viridis, Rhodoseudomonas sp., Rhodospirillum rubrum, Rhodospirillum sp., Thiocapsa roseopersicina, Thiocapsus sp., Variovorax paradoxus, Variovorax sp., Xanthobacter sp., Hydrogenothermus marinus, Ralstonia eutropha, Cupriavidus necator, Alcaligenes eutrophus, Alcaligenes paradoxus, Paracoccus denitrificans, Pseudomonas facilis, Pseudomonas flava, Pseudomonas palleronii, Pseudomonas saccharophila, and Rhodococcus sp. (Nocardia opaca), Pseudomonas pseudoflava.* In another preferred embodiment, the knallgas bacterium is a bacterium genetically modified to be a knallgas bacterium, whilst the corresponding wild type strain is not. In this case, the bacterium may be any organism amenable to such modifications, for example *E. coli.*

Many bacteria that may be used to solve the problem underlying the present invention are capable of synthesising polyhydroxybutyrate, a compound used to store carbon taken up by the cell. Such bacteria employ one or more polyhydroxybutyrate synthases. In a preferred embodiment, the term "polyhydroxybutyrate synthase", as used herein, refers to an enzyme capable of synthesising polyhydroxyalkanoates by catalysing the polymerisation of medium-chain acyl-CoA with a chain length of 3-14 carbon atoms, preferably hydroxybutyrate CoA.

In preferred embodiment, the inventive bacterium is genetically modified such that it has, compared to the respective wild type strain, a reduced capacity to synthesise polyhydroxybutyrate, or a capacity to synthesise polyhydroxybutyrate reduced such that the cell is no longer capable of synthesising detectable quantities of polyhydroxybutyrate. In a preferred embodiment, at least one gene, preferably all genes encoding a polyhydroxybutyrate synthase are knocked out, for example by introducing stop codons into the reading frame to the effect that an incomplete inactive protein is expressed.

In a preferred embodiment, the term "2-hydroxyisobutyryl-coenzyme A mutase", as used herein, refers to an enzyme capable of catalysing the formation of 2-hydroxyisobutyryl-coenzyme A, preferably by conversion of 3-hydroxybutyric coenzyme A to 2-hydroxyisobutyryl-coenzyme A:

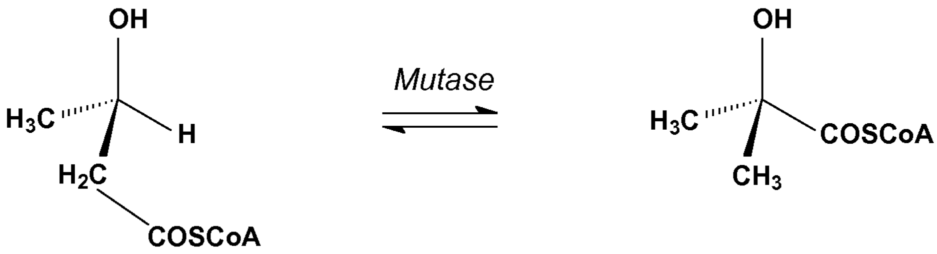


(*R*)-3-Hydroxybutyryl-CoA 2-Hydroxyisobutyryl-CoA

Such enzymes as well as organisms expressing them are disclosed in international patent applications PCT/EP2007/052830, PCT/EP2009/055089 and PCT/EP2010/065151. The mutase is a heterodimeric enzyme comprising a large substrate-binding subunit and a small coenzyme B12-binding subunit. In a preferred embodiment, the mutase is expressed by way of expression of SEQ ID NO 1 or a variant thereof and the polypeptide SEQ ID NO 2 or a variant.

In a preferred embodiment, the "2-hydroxyisobutyryl-coenzyme A mutase" is heterologous and/or overexpressed in the bacterium, i.e. expressed such that its concentration inside the cell is higher than in the respective wild type cell. In a preferred embodiment, the 2-hydroxyisobutyryl-coenzyme A mutase is expressed inside the bacterium or such that its active centre is exposed to the inside of the bacterium.

The term "2-hydroxyisobutyric acid", as any compound referred to throughout this application, includes, as used herein, not only to the protonated form, but also to any dissociated state or any salt of the compound, for example the sodium or potassium salts.

The activity of the 2-hydroxyisobutyryl-coenzyme A mutase may be increased by coexpression of a chaperone instrumental in keeping the mutase in an active conformation. In a preferred embodiment, the knallgas bacterium or acetogenic bacterium expresses, in addition to a 2-hydroxyisobutyryl-coenzyme A mutase, a MeaB protein, preferably SEQ ID NO 3, or a variant thereof, in particular a fusion comprising a MeaB protein or a variant thereof and a 2-hydroxyisobutyryl-coenzyme A mutase or a variant thereof. Suitable MeaB proteins and fusion proteins comprising MeaB as well as variants thereof are disclosed in PCT/EP2010/065151. Exemplary MeaB proteins include SEQ ID NO 3 (*Aquincola tertiaricarbonis* DSM 18512), YP_001023545 (*Methylibium petroleiphilum* PM1), YP_001409454 (*Xanthobacter autotrophicus* Py2), YP_001045518 (*Rhodobacter sphaeroides* ATCC 17029), YP_002520048 (*Rhodobacter sphaeroides*), AAL86727 (*Methylobacterium extorquens* AMI), CAX21841 (*Methylobacterium extorquens* DM4), YP_001637793 (*Methylobacterium extorquens* PA1), AAT28130 (*Aeromicrobium erythreum*), CAJ91091 (*Polyangium cellulosum*), AAM77046 (*Saccharopolyspora erythraea*), NP_417393 (*Escherichia coli* str. K-12 substr. MG1655) and variants thereof. Any access code used throughout this application refers to the respective sequence from the Genbank database run by the NCBI, wherein the release referred to is the one online on the 15th May, 2012.

The teachings of the present invention may not only carried out using biological macromolecules having the exact amino acid or nucleic acid sequences referred to in this application explicitly, for example by name or accession number, or implicitly, but also using variants of such sequences. In a preferred embodiment, the term "variant", as used herein, comprises amino acid or nucleic acid sequences, respectively, that are 70, 75, 80, 85, 90, 92, 94, 95, 96, 97, 98 or 99 % identical to the reference amino acid or nucleic acid sequence, wherein preferably amino acids other than those essential for the function, for example the catalytic activity of a protein, or the fold or structure of a molecule are deleted, substituted or replaced by insertions or essential amino acids are replaced in a conservative manner. The state of the art comprises algorithms that may be used to align two given nucleic acid or amino acid sequences and to calculate the degree of identity, see Arthur Lesk (2008), Introduction to bioinformatics, 3rd edition, Thompson et al., Nucleic Acids Research 22, 4637-4680, 1994, and Katoh et al., Genome Information, 16(1), 22-33, 2005. The term "variant" is used synonymously and interchangeably with the term "homologue". Such variants may be prepared by introducing deletions, insertions or substitutions in amino acid or nucleic acid sequences as well as fusions comprising such macromolecules or variants thereof. In a preferred embodiment, the term "variant", with regard to amino acid sequence, comprises, preferably in addition to the above sequence identity, amino acid sequences that comprise one or more conservative amino acid changes with respect to the respective reference or wild type sequence or comprises nucleic acid sequences encoding amino acid sequences that comprise one or more conservative amino acid changes. In a preferred embodiment, the term "variant" of an amino acid sequence or nucleic acid sequence comprises, preferably in addition to the above degree of sequence identity, any active portion and/or fragment of the amino acid sequence or nucleic acid sequence, respectively, or any nucleic acid sequence encoding an active portion and/or fragment of an amino acid sequence. In a preferred embodiment, the term "active portion", as used herein, refers to an amino acid sequence or a nucleic acid sequence, which is less than the full length amino acid sequence or codes for less than the full length amino acid

sequence, respectively, wherein the amino acid sequence or the amino acid sequence encoded, respectively retains at least some of its essential biological activity. For example an active portion and/or fragment of a protease is capable of hydrolysing peptide bonds in polypeptides. In a preferred embodiment, the term "retains at least some of its essential biological activity", as used herein, means that the amino acid sequence in question has a biological activity exceeding and distinct from the background activity and the kinetic parameters characterising said activity, more specifically $k_{cat}$ and $K_M$, are preferably within 3, more preferably 2, most preferably one order of magnitude of the values displayed by the reference molecule with respect to a specific substrate. In a preferred embodiment, the term "variant" of a nucleic acid comprises nucleic acids the complementary strand of which hybridises, preferably under stringent conditions, to the reference or wild type nucleic acid. Stringency of hybridisation reactions is readily determinable by one of ordinary skilled in the art, and in generally is an empirical calculation dependent on probe length, washing temperature and salt concentration. In general longer probes require higher temperatures for proper annealing, while shorter probes need lower temperatures. Hybridisation generally depends on the ability of denatured DNA to reanneal to complementary strands are present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see F. M. Ausubel (1995), Current Protocols in Molecular Biology. John Wiley & Sons, Inc.,. Moreover, the person skilled take in the art may follow the instructions given in the manual "The DIG System Users Guide for Filter Hybridization", Boehringer Mannheim GmbH, Mannheim, Germany, 1993 and in Liebl et al. (International Journal of Systematic Bacteriology 41: 255-260 (1991) on how to identify DNA sequences by means of hybridisation. In a preferred embodiment, stringent conditions are applied for any hybridisation, i.e. hybridisation occurs only if the probe is 70 % or more identical to the target sequence. Probes having a lower degree of identity with respect to the target sequence may hybridise, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50°C - 68°C, approximately 52°C - 68°C, approximately 54°C - 68°C, approximately 56°C - 68°C, approximately 58°C - 68°C, approximately 60°C - 68°C, approximately 62°C - 68°C, approximately 64°C - 68°C, approximately 66°C - 68°C. In a particularly preferred embodiment, the temperature is approximately 64°C - 68°C or approximately 66°C - 68°C. It is possible to adjust the concentration of salt to 0.2 x SSC or even 0.1 x SSC. Polynucleotide fragments having a degree of identity with respect to the reference or wild type sequence of at least 70, 80, 90, 91, 92, 93, 94, 95, 96, 97, 98, 99 % may be isolated. In a preferred embodiment, the term "homologue" of a nucleic acid sequence, as used herein, refers to any nucleic acid sequence that encodes the same amino acid sequence as the reference nucleic acid sequence, in line with the degeneracy of the genetic code.

In a preferred embodiment, the activities of 2-hydroxyisobutyryl-coenzyme A mutase, MeaB proteins, fusion proteins comprising MeaB or variants thereof may be increased relative the wild type cell from which they originate. Techniques that may be used to genetically modify bacterial cells are described in the prior art, for example in Sambrook et al. (Molecular Cloning - A Laboratory Manual (1989) Cold Spring Harbor Laboratory Press), as are methods for increasing the activity of an enzyme in a bacterial cell, for example by increasing expression of the gene encoding the enzyme having the activity of interest by way of chromosomal gene amplification (WO 03/014330 and WO 03/040373). Additional techniques that may be used to delete a gene encoding an enzyme or reduce the activity of such an enzyme in a microorganism include the exposition of cells to radioactivity followed by accumulation or screening of the resulting mutants, site-directed introduction of point mutations or knock out of a chromosomally integrated gene encoding for an active enzyme.

In a preferred embodiment, the acetogenic bacterium or Knallgas bacterium is capable of synthesizing 3-Hydroxybutyryl-Coenzym A. The latter compound may be formed in a reaction starting with acetyl-CoA catalysed by the combination of β-ketothiolase and acetoacetyl-CoA reductase. In a preferred embodiment, the term "β-ketothiolase", as used herein, refers to an enzyme capable of catalysing the conversion of two molecules acetyl-CoA to acetoacetyl-CoA and HS-CoA. An exemplary enzyme is the beta-ketothiolase from *Ralstonia eutropha* H16 (Accession number NC 008313.1). In a preferred embodiment, the term "acetoacetyl-CoA reductase", as used herein, refers to an enzyme capable of catalysing the conversion from acetoacetyl-CoA and NADPH as well as $H^+$ to 3-Hydroxybutyryl-CoA and $NADP^+$. An exemplary enzyme is the acetoacetyl-CoA reductase from *Ralstonia eutropha* H16 (Accession number NC 008313.1). A range of Knallgas bacteria and acetogenic bacteria, for example Ralstonia are capable of using this pathway using endogenous enzymes. Recombinant enzymes may be expressed in other strains such as *E. coli* to enable them to produce 3-Hydroxybutyryl-Coenzym A.

Sought-after biotechnological products used to synthesise complex polymers include not only 2-hydroxyisobutyric acid, but also derivatives thereof, in particular alkyl esters such as methyl esters. In a preferred embodiment, the inventive bacterium expresses not only a 2-hydroxyisobutyryl-coenzyme A mutase, but also an enzyme capable of catalysing the esterification of 2-hydroxyisobutyryl-coenzyme A and/or 2-hydroxyisobutyric acid. In a preferred embodiment, the enzyme capable of catalysing the esterification of 2-hydroxyisobutyryl-coenzyme A and/or 2-hydroxyisobutyric

acid is selected from the group comprising fatty acid O-methyltransferase (EC 2.1.1.15), Jasmonate O-methyltransferase (EC 2.1.1.141), Juvenile hormone O-methyltransferase (EC 2.1.1.-), Loganate O-methyltransferase (EC 2.1.1.50), alcohol O-acyltransferase (EC 2.3.1.75, EC 2.3.1.84), Acyl-CoA (Coenzyme A) thioesterase (EC 3.1.2.2, EC 3.1.2.18, EC 3.1.2.19, EC 3.1.2.20, EC 3.1.2.22) and Acyl-ACP (Acyl Carrier Protein) thioesterase (EC 3.1.2.14, EC 3.1.2.22). In a preferred embodiment, the term "fatty acid O-methyltransferase (EC 2.1.1.15)", as used herein, refers to an enzyme capable of catalyzing the reaction: S-adenosyl-L-methionine + a fatty acid = S-adenosyl-L-homocysteine + a fatty acid methyl ester. In another preferred embodiment, the term "jasmonate O-methyltransferase (EC 2.1.1.141)", as used herein, refers to an enzyme capable of catalyzing the main reaction: S-adenosyl-L-methionine + jasmonate = S-adenosyl-L-homocysteine + jasmonate methylester. IN another preferred embodiment, the term "juvenile hormone O-methyltransferase (EC 2.1.1.-)" as used herein, is an enzyme capable of catalyzing the reaction: S-adenosyl-L-methionine + juvenile hormone II acid = S-adenosyl-L-homocysteine + juvenile hormone II acid methylester. In a preferred embodiment, the term "loganate O-methyltransferase (EC 2.1.1.50)", as used herein, refers to an enzyme capable of catalyzing the reaction: S-adenosyl-L-methionine + loganate = S-adenosyl-L-homocysteine + loganate methylester. In a preferred embodiment, the term "alcohol O-acyltransferase" (EC 2.3.1.75, EC 2.3.1.84)", as used herein, catalyzing the reaction of an acyl-CoA thioester + alcohol = a fatty acid alkyl ester + CoA. In a preferred embodiment, the term "Acyl-CoA (Coenzyme A) thioesterase (EC 3.1.2.2, EC 3.1.2.18, EC 3.1.2.19, EC 3.1.2.20, EC 3.1.2.22)", as used herein, refers to an enzyme capable of catalyzing the reaction: acyl-CoA thioester + alcohol = a fatty acid alkyl ester + CoA. In a preferred embodiment, the term "Acyl-ACP (Acyl Carrier Protein) thioesterase (EC 3.1.2.14, EC 3.1.2.22))", as used herein, refers to an enzyme capable of catalyzing the reaction: acyl-ACP thioester + alcohol = a fatty acid alkyl ester + ACP.

Such a bacterium may be used to carry out the inventive method. Accordingly, the inventive method may comprise an additional step

b) alkylating, preferably methylating any 2-hydroxyisobutyric acid or 2-hydroxyisobutyryl-coenzyme A produced in step a).

Rather than using a cell coexpressing an enzyme capable of catalysing the esterification of 2-hydroxyisobutyryl-coenzyme A and/or 2-hydroxyisobutyric acid, it may be advantageous to enrich or isolate any 2-hydroxyisobutyric acid or 2-hydroxyisobutyryl-coenzyme A produced in step a) and alkylating, preferably methylating it, for example by using synthetic approaches such as acid-catalysed esterification in the presence of methanol or by contacting the compound with an enzyme having methylating activity.

The inventive method contemplates contacting the knallgas bacterium or acetogenic bacterium with a gas mixture comprising hydrogen and carbon dioxide, wherein the gas mixture comprises, in order of increasing preference, 10 to 80, 15 to 70, 20 to 60 and 25 to 50 % hydrogen and, in order of increasing preference 5 to 60, 10 to 45 and 15 to 30 % carbon dioxide, with the proviso that the sum of the volumes is no more than 100 %, preferably at atmospheric pressure.

If the bacterium used to carry out is an aerotolerant bacterium, the gas mixture may comprise oxygen in addition to hydrogen and carbon dioxide. In a preferred embodiment, the gas mixture comprises, in order of increasing preference, up to 20, 15, 10, 5 or 1 % oxygen. In a preferred embodiment, the term "aerotolerant", as used herein, means that the bacterium of interest is capable of growing in the presence of up to 1, 5, 10, 15 or 20 % oxygen, in order of increasing preference, preferably at atmospheric pressure The person skilled in the art is able to readily determine whether or not a bacterium is aerotolerant, for example by growing that bacterium under a gas atmosphere comprising a defined amount of oxygen and monitoring whether any growth occurs as indicated, for example, by a change of optical density or light scattering of the medium used.

The pressure of the gas mixture applied is, in a preferred embodiment, 0.5 to 10 bars, more preferably 0.8 to 8, even more preferably 1.5 to 6 bar. In another preferred embodiment, the pressure is more than 1, 1.5, 2, 3, 4, 5, 6, 7, 8, 9, 10 bar. In a preferred embodiment, the pressure applied exceeds atmospheric pressure. In a preferred embodiment, the hydrogen partial pressure is 0.1 to 100 bar, more preferably 0.2 to 10 bar, most preferably 0.5 to 4 bar. In a preferred embodiment, the carbon dioxide partial pressure is 0.03 to 100 bar, more preferably 0,05 to 1 bar, most preferably 0.05 to 0.3 bar. In a preferred embodiment, the oxygen partial pressure is 0.03 to 10 bar, more preferably 0.04 to 1 bar, most preferably 0.04 to 0.5 bar. In a preferred embodiment, the total combined pressure is 1 to 200 bar, preferably 1 to 10 bar, most preferably 1 to 5 bar.

In a preferred embodiment, the knallgas bacterium or acetogenic bacterium has a reduced fatty acid degradation capacity. In a preferred embodiment, the term "having a reduced fatty acid degradation capacity", as used in herein, means that the respective microorganism degrades fatty acids, preferably those taken up from the environment, at a lower rate than a comparable microorganism having normal fatty acid degradation capacity would. In a preferred embodiment, the fatty acid degradation of such a microorganism is lower on account of deletion, inhibition or inactivation of at least one gene encoding an enzyme involved in the β-oxidation pathway. In a preferred embodiment of the present invention, at least one enzyme involved in the β-oxidation pathway has lost, in order of increasing preference, 5, 10, 20,

40, 50, 75, 90 or 99 % activity relative to the activity of the same enzyme under comparable conditions in the respective wild type microorganism. The person skilled in the art is familiar with various techniques that may be used to delete a gene encoding an enzyme or reduce the activity of such an enzyme in a microorganism, for example by exposition of cells to radioactivity followed by accumulation or screening of the resulting mutants, site-directed introduction of point mutations or knock out of a chromosomally integrated gene encoding for an active enzyme, as described in Sambrook/Fritsch/Maniatis (1989). In addition, the transcriptional repressor FadR may be over expressed to the effect that expression of enzymes involved in the β-oxidation pathway is repressed (Y Fujita, H Matsuoka, and K Hirooka (2007) Mol. Microbiology 66(4), 829-839). In a preferred embodiment, the term "deletion of a gene", as used herein, means that the nucleic acid sequence encoding said gene is modified such that the expression of active polypeptide encoded by said gene is reduced. For example, the gene may be deleted by removing in-frame a part of the sequence comprising the sequence encoding for the catalytic active centre of the polypeptide. Alternatively, the ribosome binding site may be altered such that the ribosomes no longer translate the corresponding RNA. Moreover, the person skilled in the art is able to routinely measure the activity of enzymes expressed by living cells using standard essays as described in enzymology text books, for example A Cornish-Bowden (1995), Fundamentals of Enzym Kinetics, Portland Press Limited, 1995. The state of the art discloses various tests designed specifically for determining the activity of enzymes involved in the β-oxidation pathway, for example K Kameda & W D Nunn (1981) J. Biol. Chem. 256, 5702-5707, H Marrakchi, W E DeWolf, C Quinn, J West, B J Polizzi, C Y So et al. (2003) Biochem. J. 370, 1055-1062, S Lobo, G Florova, and K A Reynolds (2001) Biochemistry 40 (39), 11955-64, X Yu, T Liu, F Zhu, and C Khosla (2011) PNAS, published online*)*.

According to the inventive method, the acetogenic bacterium or knallgas bacterium is contacted with the gas mixture in an aqueous medium. In a preferred embodiment, the term "aqueous medium" comprises any aqueous solution that comprises the amount of salts and buffers necessary to grow or sustain an acetogenic bacterial cell and to sustain acetogenesis. For example, an aqueous medium according to Hurst, K. M., and Lewis, R. (2010), Biochemical Engineering Journal 2010, 48, 159-165 may be used to carry out the inventive teachings.

The temperature in steps a) and b) needs to be chosen bearing in mind the needs of the acetogenic bacterium or knallgas bacterium on the one hand and thermodynamic parameters on the other hand. The state of the art teaches ranges of temperatures as well as optimum temperatures for a vast range of acetogenic bacteria. For example, *Clostridium thermoaceticum* may be incubated at temperatures of up to 60 °C. See also standard textbooks of microbiology for temperatures that may be used to grow acetogenic bacterial and archaeal cells, for example Dworkin et al. (2006) The Prokaryotes - A Handbook on the Biology of Bacteria, Volume 2. In a preferred embodiment, the temperature applied in step a) is, in order of increasing preference, 0 to 100 °C, 10 to 80 °C, 20 to 60 °C, 30 to 45 °C or 35 to 42 °C. In another preferred embodiment, the temperature applied in step a) is 37 °C or more.

The invention is further illustrated by the following figures and non-limiting examples from which further features, embodiments, aspects and advantages of the present invention may be taken.

Fig. 1 shows calculated data for hydrogen conversion efficiency out of biomass concentrations of 0-60 g/L and 2-HIB product concentrations of 0-100 g/L.

Fig. 2 shows experimental data obtained when carrying out Example 1, more specifically the growth phase (0-60 h) and product synthesis phase (60 h-160 h) with • biomass in g/L ☆ 2-HIB in g/L and pH.

**Example 1: Biotechnological production of 2-hydroxyisobutyric acid using a genetically modified strain of *Cupriavidus necator* H16 PHB-4 [47] DSM 541**

**METHODS**

**Bacterial strains and plasmids**

*Cupriavidus necator* H16 PHB-4 [47] DSM 541 was obtained from the DSMZ (Leibniz-Institut DSMZ - Deutsche Sammlung von Mikroorganismen und Zellkulturen GmbH, Braunschweig, Germany) and modified by introducing the plasmid pBBR1MCS-2::HCM (Reinecke L, Schaffer S, Marx A, Pötter M, Haas T (2009) Recombinant cell producing 2-hydroxyisobutyric acid. WO/2009/156214), which originates from broad host range cloning vector pBBR1MCS (Kovach ME, Elzer PH, Hill DS, Robertson GT, Farris MA, Roop RM, Peterson KM (1995) Four new derivatives of the broad-host-range cloning vector pBBR1MCS, carrying different antibiotic-resistance cassettes. Gene 166(1):175-6).. The plasmid contains the genes *hcmA* and *hcmB* coding for the two subunits of the 2-hydroxy-isobutyryl-coenzyme A mutase from *Aquincola tertiaricarbonis* (Yaneva N, Schuster J, Schäfer F, Lede V, Przybylski D, Paproth T, Harms H, Müller RH, Rohwerder T (2012) A bacterial acyl-CoA mutase specifically catalyzes coenzyme B12-dependent isomerization of 2-hydroxyisobutyryl-CoA and (S)-3-hydroxybutyryl-CoA. J Biol Chem. doi:10.1074/jbc.M111.314690).

**Cultivation conditions**

General cultivation was done in Luria Bertani broth (Miller) at 30 °C and the strain was stored on LB-agar plates at 4°C. For batch and continuous cultivations a mineral salt medium was used as described by Schlegel and co-authors (Schlegel HG, Kaltwasser H, Gottschalk G (1961) Ein Submersverfahren zur Kultur wasserstoffoxydierender Bakterien: Wachstumsphysiologische Untersuchungen. Arch Microbiol 38(3):209-222) supplied with kanamycin. Cultivations were carried out in lab-scale with shaking flasks containing 0.6 L working volume at 22 °C. Agitation was set to 200 rpm and aeration was kept constant at 5 L/h.

Pre-cultures were prepared from single colonies at 30 °C and 150 rpm in 200 mL of the same medium but fructose as sole carbon source substrate and under aerobic conditions. After fructose exhaustion the culture was shifted to hydrogen and carbon dioxide and cultivation continued under batch conditions. The two gases in addition to oxygen were supplied from a storage tank of 18 L treated according to the gasometer principle. The initial gas concentrations were about 25-50 % H2, 15-30% CO2 and 10-20 % 02. Gases were offered to the culture via a hollow fiber module (Fresenius, St. Wendel, Germany) by a membrane pump at rate of 750 L/h. Hollow fibers had a pore width of 0.2 µm and a specific exchange area of 0.7 $m^2$. The external volume of the hollow fiber module was flushed with the bacterial suspension at a rate of 750 L/h, fed with a gear pump. After passage through the module, gases and suspension were carried in a flask equipped with a stirrer. Gases were re-circulated to the gas tank and mixed with the residing gases by a propeller by means of a magnet-coupled motor installed outside of the tank. Consumption of gases was monitored in terms of total volume registering the horizontal movement of the gas tank and in terms of concentration by three specific sensors. Furthermore, gas consumption was calculated from the differential changes of total gas and individual gas concentrations. If required specific gases were refilled to the gas tank. As there was no automated pH control in this simplified cultivation system, pH was monitored off-line and adjusted to pH 7.5 by adding required volumes of 10 % NaOH according to a titration curve based on the growth medium.

**On-line analysis**

Gas concentrations were measured by specific sensors for hydrogen (0-100 %), oxygen (0-50 %) and carbon dioxide (0-50 %) (BlueSens, Herten, Germany) and their concentrations continuously monitored. Tubing of the cultivation system was arranged in a way that allowed measuring the gases from inflow as well as outflow.

**Off-line analysis**

Biomass concentration was determined by optical density at 700 nm and gravimetrically in quadruplicate after oven-drying at 105 °C. Substrate consumption and 2-HIB synthesis were analyzed by isocratic HPLC (Shimadzu) on a Nucleogel Ion 3000A column (300mm×7.8mm, Macherey-Nagel GmbH & Co. KG, Düren, Germany) at 70 °C with 0.6 mL/min 0.01 N $H_2SO_4$ as eluant.

**Calculations:**

3-phosphoglycerate (PGA) was defined as the central carbon precursor [51, 52] from which the complete biomass synthesis was derived. The molar composition of biomass in the model was taken as $C_4H_8O_2N$. It is synthesized from ATP as the general energy carrier and proceeds with an efficiency of 10.5 g dry mass pro mol ATP (Stouthamer AH (1973) A theoretical study on the amount of ATP required for synthesis of microbial cell material. Antonie Van Leeuwenhoek 39(3):545-65). The overall balance equation for biomass synthesis from PGA is accordingly:

$$4\ PGA + 29.1\ ATP + 3\ NH_3 + 5.5\ [2H] \rightarrow 3\ C_4H_8O_2N + 10\ H_2O \qquad [1]$$

[2H] stands for reduction equivalents which correspond in general to NAD(P)H + $H^+$.

**RESULTS AND DISCUSSION**

To define possible product yields in a growth associated process we implied a stoichiometric model. Knallgas bacteria like C. *necator* use the Calvin cycle to assimilate carbon and the enzyme hydrogenase to gain NAD(P)H + $H^+$ from hydrogen as substrate for oxidative phosphorylation via the respiratory chain as well as source for carbon dioxide reduction. Therefore, the overall balance equation for biomass synthesis via PGA including energy generation from $H_2$

oxidation at P/O = 2 results in

$$12\ CO_2 + 3\ NH_3 + 56.05\ [H_2] + 15.28\ O_2 \rightarrow 3\ C_4H_8O_2N + 48.55\ H_2O \qquad \text{(eq. 2)}$$

With respect to growth this corresponds to a carbon conversion efficiency of CCE of 1 Cmol incorporated per Cmol supplied and a HCE (hydrogen conversion efficiency) of 0.214 Hmol assimilated per Hmol consumed. Synthesis of 2-HIB ($C_4H_8O_3$) as desired product via the Calvin cycle with PGA and pyruvate as intermediates results in acetyl-CoA (AcCoA) according to

$$4\ CO_2 + 8\ [H_2] + 14\ ATP \rightarrow 2\ AcCoA + 4\ H_2O \qquad \text{(eq. 3a)}$$

The ATP required for $CO_2$ fixation is obtained from hydrogen oxidation via the respiratory chain; accordingly eq. 3a is extended to

$$4\ CO_2 + 16\ [H_2] + 3.5\ O_2 \rightarrow 2\text{-HIB} + 12\ H_2O \qquad \text{(eq. 3b)}$$

The CCE is again 1 Cmol/Cmol, whereas the theoretical HCE is 0.25 Hmol/Hmol (eq. 3b). Combining biomass synthesis and product formation to an integral process, the interdependency between both processes defining the final HCE with respect to product is shown in Figure 1. We took into account two ranges of biomass concentration extending from 0 to 10 g/L and from 10 to 60 g/L to consider a wide spectrum of variables. Obviously, biomass synthesis is very costly (eq. 2). It is apparent that the overall process approaches a value of 0.2 to 0.25 Hmol/Hmol when the biomass concentration is below 10 g/L and the product concentration moves towards 100 g/L (Figure 1). Both, the increase in biomass and the reduction of product concentration, drastically decrease the HCE.

We used the strain C. *necator* H16 PHB-4 (Schlegel HG, Lafferty R, Krauss I (1970) The isolation of mutants not accumulating poly-β-hydroxybutyric acid. Arch Microbiol 71(3):283-294), which is deficient in PHB synthesis due to knock-out of the PHB polymerase gene. This allowed this strain to synthesize metabolites up to 3-HB-CoA under conditions of overflow metabolism. Cloning and expressing the 2-HIB-CoA mutase from *A. tertiaricarbonis* established the alternative/additional route for our desired dead-end product, 2-HIB. To proof the capacity of the chosen system for 2-HIB synthesis the strain, pre-grown on fructose, was used to inoculate the cultivation apparatus. A gas stream containing 25-50 % $H_2$, 15-30 % $CO_2$ and 10-20 % $O_2$ was supplied as growth substrate therefore resulting in the induction of the enzymes required for chemo-litho-autotrophic growth, especially hydrogenases (Lenz O, Schwartz E, Dernedde J, Eitinger M, Friedrich B (1994) The Alcaligenes eutrophus H16 hoxX gene participates in hydrogenase regulation. J Bacteriol 176(14):4385-93; Burgdorf T, Lenz O, Buhrke T, van der Linden E, Jones AK, Albracht SP, Friedrich B (2005) [NiFe]-hydrogenases of Ralstonia eutropha H16: modular enzymes for oxygen-tolerant biological hydrogen oxidation. J Mol Microbiol Biotechnol 10(2-4):181-96) and for carbon dioxide fixation (Husemann M, Klintworth R, Büttcher V, Salnikow J, Weissenborn C, Bowien B (1988) Chromosomally and plasmid-encoded gene clusters for CO2 fixation (cfx genes) in Alcaligenes eutrophus. Mol Gen Genet MGG 214(1):112-120)). Under these conditions growth preceded at a rate of about 0.058/h until the nitrogen source was exhausted, attaining a final biomass concentration of approx. 2.0 g/L (Figure 2). During exponential growth carbon dioxide was incorporated into biomass with a CCE = 0.58 Cmol/Cmol. Hydrogen conversion yielded an HCE of 0.0715 Hmol/Hmol. It should be noted that the theoretical maximum possible value of HCE with 0.214 Hmol/Hmol can not be attained due to requirements of energy ($H_2$) necessary for maintenance purposes. Larger deviations from the theoretical values might be caused by the synthesis of products other than biomass, such as pyruvate. More detailed analyses, however, were not undertaken at this stage of investigation.

During biomass growth 2-HIB was found of only at low concentration. After exhaustion of the nitrogen source, however, there was a steep increase in external 2-HIB concentration (Figure 2). The synthesis rate corresponded to 8.35 mg 2-HIB/(g dry mass h). This rate was stable up to a concentration of approx. 410 mg/L. Subsequently the product synthesis rate suddenly ceased which was likewise observed in repeated experiments. The abrupt shift indicated a distinct limitation/disturbance not obvious from the experimental setup but requiring further optimization. The amount of gases consumed per increment of 2-HIB was used to calculate the yield coefficients. The data was corrected for unspecific

loss of gases determined by running experiments in the absence of biomass. The remaining was incorporated into 2-HIB with a CCE of 0.178 Cmol/Cmol. Hydrogen as second substrate was converted into this product with a HCE of 0.032 Hmol/Hmol. Noticeably, this is far from the above stated limit values.

The deficit in the balance of substrates can likely be explained by the putative synthesis of products other than 2-HIB. Taking into account the reduction of $CO_2$ by hydrogen to yield the first intermediate of carbon fixation in the Calvin cycle, glyceraldehyde-3-phosphate (GAP, $C_3H_6O_3$, phosphate-free sum formula), the $CO_2$ available due to the present consumption characteristic will allow the synthesis of 0.57 mmol GAP per hour in the linear phase of 2-HIB formation. This in turn requires 5.16 mmol $H_2$/h based on

$$3\ CO_2 + 9\ [H_2] + 1.5\ O_2 \text{->} GAP\ (C_3H_6O_3) + 6\ H_2O \qquad \text{(eq. 4)}$$

Due to the hydrogen balance 5.71 mmol $H_2$ remain available after 2-HIB synthesis which could satisfy the putative product synthesis. Moreover, some hydrogen further remains available for maintenance purposes which are inevitably necessary in living cells. Taking into account the specific maintenance coefficient determined formerly for *Ralstonia eutropha* (*C. necator*) JMP 134 on fructose of $m_s$ = 0.09 mmol/(g dry mass h) [57] and converting this substrate-based coefficient into an energy (ATP)-based value (P/O=2) which would be equivalent to $m_e$ = 2.34 mmol ATP/(g dry mass h) the hydrogen remaining after 2-HIB synthesis and putative other reduced products would be sufficient to generate 2.8 mmol ATP/(g dry mass h). This is in pretty coincidence with former results to this species.

## Claims

1. A knallgas bacterium or acetogenic bacterium expressing a 2-hydroxyisobutyryl-coenzyme A mutase.

2. The knallgas bacterium or acetogenic bacterium according to claim 1, wherein the bacterium is genetically modified such that it has, compared to the respective wild type strain, a reduced capacity to synthesise polyhydroxybutyrate.

3. The knallgas bacterium or acetogenic bacterium according to claim 2, wherein the bacterium has a polyhydroxybutyrate synthase gene knockout.

4. The knallgas bacterium or acetogenic bacterium according to any of claims 1 to 3, wherein the bacterium is incapable of synthesising polyhydroxybutyrate.

5. The knallgas bacterium or acetogenic bacterium according to any of claims 1 to 4, wherein the bacterium is a knallgas bacterium selected from the group comprising *Methanobacterium, Acetobacterium, Desulfovibrio, Desulfomonas, Paracoccus, Achromobacter, Alcaligenes, Pseudomonas, Nocardia* and *Cupriavidus,* and is preferably a *Cupriavidus* strain, most preferably *Cupriavidus necator* H16, or an acetogenic bacterium selected from the group comprising *Clostridium, Moorella* and *Acetoanaerobium, Acetobacterium, Archaeoglobus, Butyribacterium, Carboxydibrachium, Carboxydocella, Carboxydothermus, Citrobacter, Desulfotomaculum, Eubacterium, Methanosarcina, Methanothermobacter, Oxobacter Peptostreptococcus, Rhodopseudomonas, Rhodospirillum, Rubrivivax, Thermincola, Thermococcus, Thermolithobacter, Thermoanaerobacter and Thermosyntrophicum*.

6. The knallgas bacterium or acetogenic bacterium according to any of claims 1 to 5, wherein the bacterium expresses thea nucleic acid comprising SEQ ID NO 1 or a variant thereof or a polypeptide encoded by said nucleic acid or a variant of said polypeptide and expresses a nucleic acid comprising SEQ ID NO 2 or a variant thereof or a polypeptide encoded by the latter nucleic acid or a variant of the latter polypeptide.

7. The knallgas bacterium or acetogenic bacterium according to any of claims 1 to 6, wherein the bacterium expresses, in addition to a 2-hydroxyisobutyryl-coenzyme A mutase, a MeaB protein or a variant thereof, preferably the polypeptide encoded by SEQ ID NO 3 or a variant thereof.

8. The knallgas bacterium or acetogenic bacterium according to any of claims 1 to 7, wherein the bacterium is capable of synthesizing 3-Hydroxybutyryl-Coenzym A.

9. The knallgas bacterium or acetogenic bacterium according to any of claims 1 to 8, wherein the bacterium expresses a β-ketothiolase and an acetoacetyl-CoA reductase.

10. A method for the production of 2-hydroxyisobutyric acid, comprising

    c) contacting in an aqueous medium the knallgas bacterium or acetogenic bacterium according to any of claims 1 to 9 with a gas mixture comprising hydrogen and carbon dioxide.

**11.** The method according to claim 10, wherein the hydrogen partial pressure in the gas mixture is 0.1 to 100 bar, more preferably 0.2 to 10 bar, most preferably 0.5 to 4 bar and the carbon dioxide partial pressure is 0.03 to 100 bar, more preferably 0,05 to 1 bar, most preferably 0.05 to 0.3 bar.

**12.** The method according to any of claims 10 to 11, wherein the bacterium tolerates the presence of oxygen and aqueous solution is aerobic.

**13.** The method according to any of claims 10 to 12, wherein the bacterium tolerates the presence of oxygen and the gas mixture comprises oxygen in addition to hydrogen and carbon dioxide, and the oxygen partial pressure in the gas mixture is preferably 0.03 to 10 bar, more preferably 0.04 to 1 bar, most preferably 0.04 to 0.5 bar.

**14.** The method according to any of claims 10 to 13, wherein the aqueous medium does not comprise carbohydrates or, preferably, any carbon source other than carbon dioxide.

**15.** The method according to any of claims 10 to 14, wherein step a) is carried out in the absence of a growth-limiting nutrient other than a carbon.

**16.** The method according to any of claims 10 to 15, wherein step a) is carried out at 20 to 37 °C, more preferably 25 to 35 °C, most preferably at 28 to 32 °C.

**17.** A use of the knallgas bacterium or acetogenic bacterium according to any of claims 1 to 9 for the production of 2-hydroxyisobutyric acid.

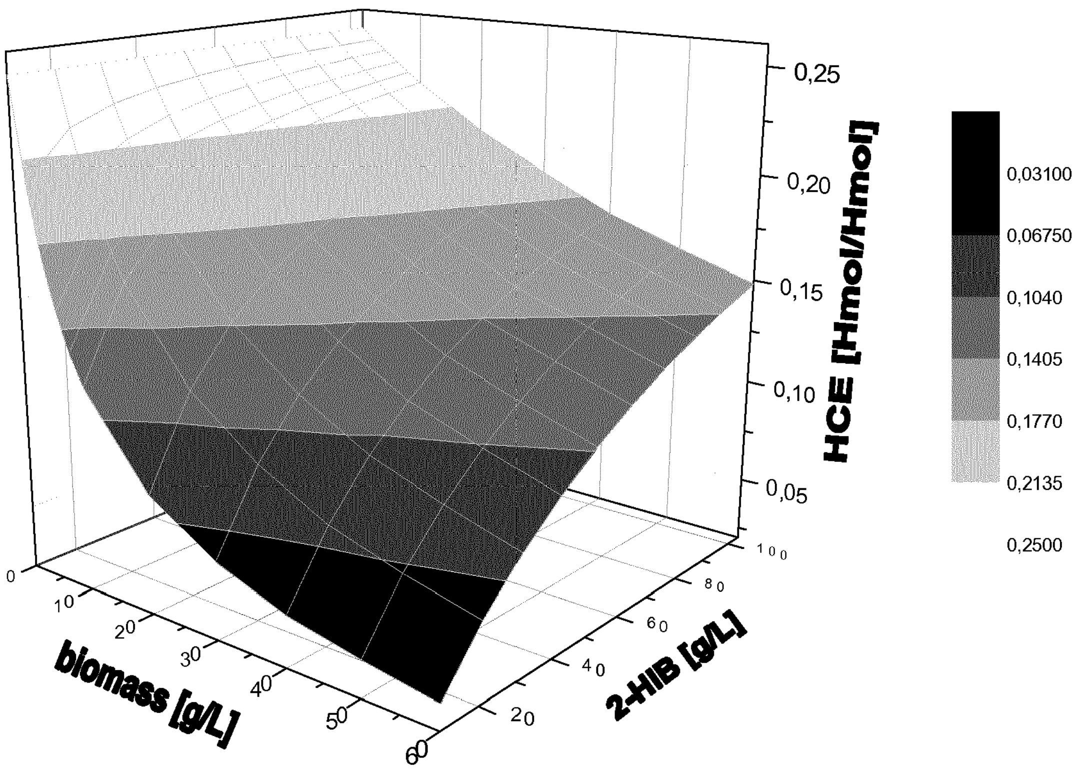
0,25
0,20
0,15
0,10
0,05
HCE [Hmol/Hmol]
0,03100
0,06750
0,1040
0,1405
0,1770
0,2135
0,2500
0
10
20
30
40
50
60
biomass [g/L]
20
40
60
80
100
2-HIB [g/L]

Fig. 1

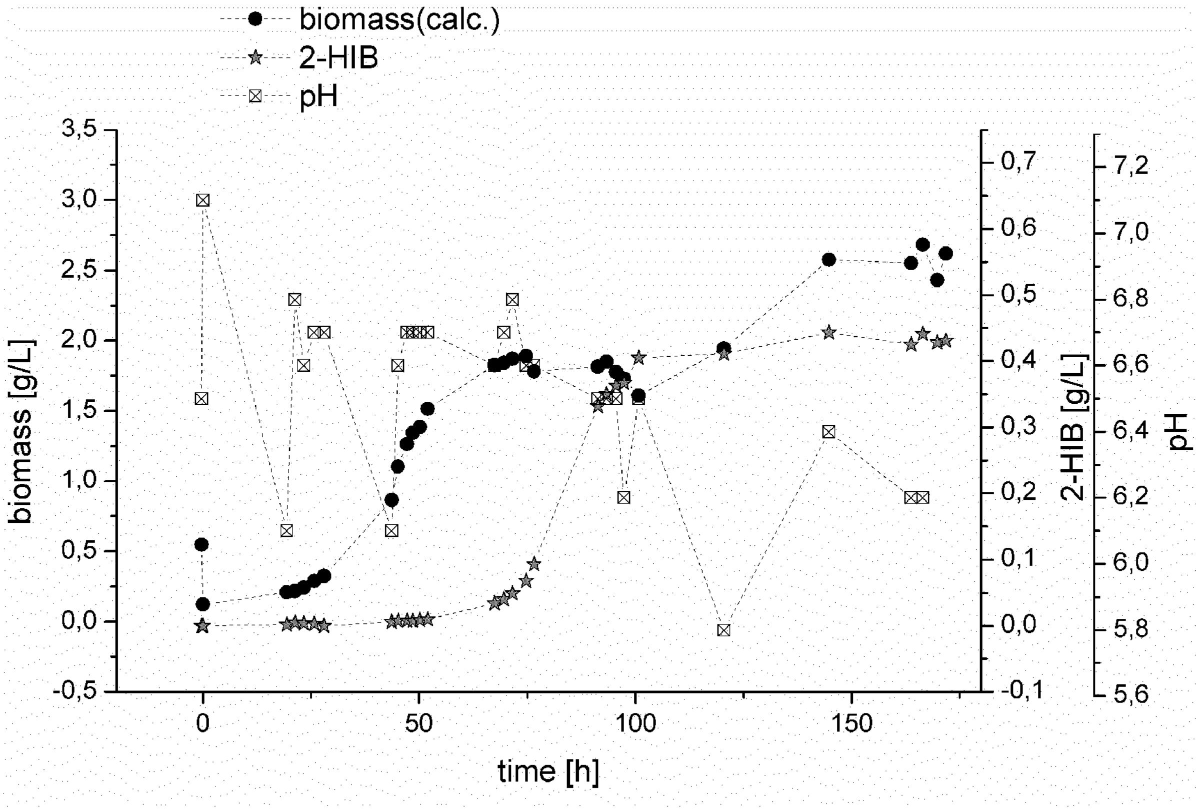
biomass(calc.)
2-HIB
pH
biomass [g/L]
2-HIB [g/L]
pH
time [h]
3,5
3,0
2,5
2,0
1,5
1,0
0,5
0,0
-0,5
0,7
0,6
0,5
0,4
0,3
0,2
0,1
0,0
-0,1
7,2
7,0
6,8
6,6
6,4
6,2
6,0
5,8
5,6
0
50
100
150

**Fig. 2**

Europäisches Patentamt
European Patent Office
Office européen des brevets

# EUROPEAN SEARCH REPORT

Application Number
EP 12 17 3010

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br><br><br>Y | WO 2009/156214 A1 (EVONIK ROEHM GMBH [DE]; REINECKE LIV [DE]; SCHAFFER STEFFEN [DE]; MARX) 30 December 2009 (2009-12-30)<br>* the whole document *<br>* page 22, paragraph 3 - page 23, paragraph 1 *<br>* examples 2-3 *<br>----- | 1-6,8-17<br><br><br>7 | INV.<br>C12N9/90<br>C12N15/63<br>C12P7/42<br>C12P7/52 |
| X,D<br><br><br>Y | WO 2011/057871 A2 (EVONIK ROEHM GMBH [DE]; REINECKE LIV [DE]; SCHAFFER STEFFEN [DE]; KOEH) 19 May 2011 (2011-05-19)<br>* the whole document *<br>* claims 14,18 *<br>* example 2 *<br>----- | 1-17<br><br><br>7 | |
| X | WO 2009/135074 A2 (GENOMATICA INC [US]; BURK MARK J [US]; BURGARD ANTHONY P [US]; OSTERHO) 5 November 2009 (2009-11-05)<br>* the whole document *<br>* figure 12 *<br>* example XI *<br>* page 32, line 12 - page 33, line 23 *<br>----- | 1,6,<br>8-11,<br>14-17 | TECHNICAL FIELDS SEARCHED (IPC) |
| Y,D | N. YANEVA ET AL: "Bacterial Acyl-CoA Mutase Specifically Catalyzes Coenzyme B12-dependent Isomerization of 2-Hydroxyisobutyryl-CoA and (S)-3-Hydroxybutyryl-CoA",<br>JOURNAL OF BIOLOGICAL CHEMISTRY,<br>vol. 287, no. 19, 4 May 2012 (2012-05-04), pages 15502-15511, XP55036888,<br>ISSN: 0021-9258, DOI: 10.1074/jbc.M111.314690<br>* the whole document *<br>-----<br>-/-- | 1-17 | C12N<br>C12P |

The present search report has been drawn up for all claims

| Place of search | Date of completion of the search | Examiner |
|---|---|---|
| Munich | 5 September 2012 | Giebeler, Katharina |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 17 3010

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2007/110394 A2 (HELMHOLTZ ZENTRUM FUER UMWELTF [DE]; MUELLER ROLAND H [DE]; ROHWERDER) 4 October 2007 (2007-10-04)<br>* the whole document *<br>----- | 1-17 | |
| Y | WO 2010/022763 A1 (METABOLIC EXPLORER SA [FR]; SOUCAILLE PHILIPPE [FR]; BOISART CEDRIC [F) 4 March 2010 (2010-03-04)<br>* the whole document *<br>----- | 1-17 | |
| A | ROHWERDER THORE ET AL: "The alkyl tert-butyl ether intermediate 2-hydroxyisobutyrate is degraded via a novel cobalamin-dependent mutase pathway", APPLIED AND ENVIRONMENTAL MICROBIOLOGY, AMERICAN SOCIETY FOR MICROBIOLOGY, US, vol. 72, no. 6, 1 June 2006 (2006-06-01), pages 4128-4135, XP002460829, ISSN: 0099-2240, DOI: 10.1128/AEM.00080-06<br>* the whole document *<br>----- | 1-17 | |
| T | VALENTIN CRACAN ET AL: "Novel B 12 -Dependent Acyl-CoA Mutases and Their Biotechnological Potential", BIOCHEMISTRY, vol. 51, no. 31, 7 August 2012 (2012-08-07), pages 6039-6046, XP55037105, ISSN: 0006-2960, DOI: 10.1021/bi300827v<br>* the whole document *<br>----- | | TECHNICAL FIELDS SEARCHED (IPC) |

1

The present search report has been drawn up for all claims

| Place of search | Date of completion of the search | Examiner |
|---|---|---|
| Munich | 5 September 2012 | Giebeler, Katharina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 12 17 3010

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2012

| Patent document cited in search report | Publication date | Patent family member(s) | Publication date |
|---|---|---|---|
| WO 2009156214 A1 | 30-12-2009 | CA 2729476 A1 | 30-12-2009 |
| | | CN 102076864 A | 25-05-2011 |
| | | DE 102008002715 A1 | 31-12-2009 |
| | | EP 2291530 A1 | 09-03-2011 |
| | | JP 2011525367 A | 22-09-2011 |
| | | KR 20110022625 A | 07-03-2011 |
| | | US 2011171702 A1 | 14-07-2011 |
| | | WO 2009156214 A1 | 30-12-2009 |
| WO 2011057871 A2 | 19-05-2011 | CA 2780529 A1 | 19-05-2011 |
| | | DE 102009046623 A1 | 12-05-2011 |
| | | DE 112010004358 A5 | 23-08-2012 |
| | | WO 2011057871 A2 | 19-05-2011 |
| WO 2009135074 A2 | 05-11-2009 | AU 2009242615 A1 | 05-11-2009 |
| | | CA 2722680 A1 | 05-11-2009 |
| | | EP 2285973 A2 | 23-02-2011 |
| | | JP 2011519561 A | 14-07-2011 |
| | | US 2009275096 A1 | 05-11-2009 |
| | | WO 2009135074 A2 | 05-11-2009 |
| WO 2007110394 A2 | 04-10-2007 | AT 467684 T | 15-05-2010 |
| | | AU 2007229522 A1 | 04-10-2007 |
| | | BR PI0709142 A2 | 28-06-2011 |
| | | CA 2653028 A1 | 04-10-2007 |
| | | DE 102006017760 A1 | 27-09-2007 |
| | | EP 1999264 A2 | 10-12-2008 |
| | | JP 2009538118 A | 05-11-2009 |
| | | KR 20090005052 A | 12-01-2009 |
| | | RU 2008142188 A | 27-04-2010 |
| | | US 2010035314 A1 | 11-02-2010 |
| | | US 2011165640 A1 | 07-07-2011 |
| | | WO 2007110394 A2 | 04-10-2007 |
| WO 2010022763 A1 | 04-03-2010 | CN 102203267 A | 28-09-2011 |
| | | JP 2012500641 A | 12-01-2012 |
| | | US 2011151530 A1 | 23-06-2011 |
| | | WO 2010022763 A1 | 04-03-2010 |
| | | WO 2010023206 A1 | 04-03-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2009055089 W
- EP 2007052830 W
- EP 2010065151 W
- WO 03014330 A
- WO 03040373 A
- WO 2009156214 A


### Non-patent literature cited in the description


- Introduction to bioinformatics. **ARTHUR LESK ; THOMPSON et al.** Nucleic Acids Research. 2008, vol. 22, 4637-4680
- **KATOH et al.** *Genome Information,* 2005, vol. 16 (1), 22-33
- **F. M. AUSUBEL.** Current Protocols in Molecular Biology. John Wiley & Sons, Inc, 1995
- The DIG System Users Guide for Filter Hybridization. 1993
- **LIEBL et al.** *International Journal of Systematic Bacteriology,* 1991, vol. 41, 255-260
- **SAMBROOK et al.** Molecular Cloning - A Laboratory Manual. Cold Spring Harbor Laboratory Press, 1989
- **Y FUJITA ; H MATSUOKA ; K HIROOKA.** *Mol. Microbiology,* 2007, vol. 66 (4), 829-839
- **A CORNISH-BOWDEN.** Fundamentals of Enzym Kinetics. Portland Press Limited, 1995
- **K KAMEDA ; W D NUNN.** *J. Biol. Chem.,* 1981, vol. 256, 5702-5707
- **H MARRAKCHI ; W E DEWOLF ; C QUINN ; J WEST ; B J POLIZZI ; C Y SO et al.** *Biochem. J.,* 2003, vol. 370, 1055-1062
- **S LOBO ; G FLOROVA ; K A REYNOLDS.** *Biochemistry,* 2001, vol. 40 (39), 11955-64
- **X YU ; T LIU ; F ZHU ; C KHOSLA.** *PNAS,* 2011
- **HURST, K. M. ; LEWIS, R.** *Biochemical Engineering Journal,* 2010, vol. 48, 159-165
- **DWORKIN et al.** The Prokaryotes - A Handbook on the Biology of Bacteria. 2006, vol. 2
- **KOVACH ME ; ELZER PH ; HILL DS ; ROBERTSON GT ; FARRIS MA ; ROOP RM ; PETERSON KM.** Four new derivatives of the broad-host-range cloning vector pBBR1MCS, carrying different antibiotic-resistance cassettes. *Gene,* 1995, vol. 166 (1), 175-6
- **YANEVA N ; SCHUSTER J ; SCHÄFER F ; LEDE V ; PRZYBYLSKI D ; PAPROTH T ; HARMS H ; MÜLLER RH ; ROHWERDER T.** A bacterial acyl-CoA mutase specifically catalyzes coenzyme B12-dependent isomerization of 2-hydroxyisobutyryl-CoA and (S)-3-hydroxybutyryl-CoA. *J Biol Chem.,* 2012
- **SCHLEGEL HG ; KALTWASSER H ; GOTTSCHALK G.** Ein Submersverfahren zur Kultur wasserstoffoxydierender Bakterien: Wachstumsphysiologische Untersuchungen. *Arch Microbiol,* 1961, vol. 38 (3), 209-222
- **STOUTHAMER AH.** A theoretical study on the amount of ATP required for synthesis of microbial cell material. *Antonie Van Leeuwenhoek,* 1973, vol. 39 (3), 545-65
- **SCHLEGEL HG ; LAFFERTY R ; KRAUSS I.** The isolation of mutants not accumulating poly-β-hydroxybutyric acid. *Arch Microbiol,* 1970, vol. 71 (3), 283-294
- **LENZ O ; SCHWARTZ E ; DERNEDDE J ; EITINGER M ; FRIEDRICH B.** The Alcaligenes eutrophus H16 hoxX gene participates in hydrogenase regulation. *J Bacteriol,* 1994, vol. 176 (14), 4385-93
- **BURGDORF T ; LENZ O ; BUHRKE T ; VAN DER LINDEN E ; JONES AK ; ALBRACHT SP ; FRIEDRICH B.** NiFe]-hydrogenases of Ralstonia eutropha H16: modular enzymes for oxygen-tolerant biological hydrogen oxidation. *J Mol Microbiol Biotechnol,* 2005, vol. 10 (2-4), 181-96
- **HUSEMANN M ; KLINTWORTH R ; BÜTTCHER V ; SALNIKOW J ; WEISSENBORN C ; BOWIEN B.** Chromosomally and plasmid-encoded gene clusters for CO2 fixation (cfx genes) in Alcaligenes eutrophus. *Mol Gen Genet MGG,* 1988, vol. 214 (1), 112-120

SEQUENCE LISTING

<110> Evonik Industries AG

<120> Biotechnological 2-hydroxyisobutyric acid production

<130> 2012E00115 DE

<160> 3

<170> PatentIn version 3.5

<210> 1
<211> 1689
<212> DNA
<213> artificial

<220>
<223> 2-hydroxyisobutyryl-coenzyme A mutase - subunit 1

<400> 1
```
atgacctggc ttgagccgca gataaagtcc caactccaat cggagcgcaa ggactgggaa      60

gcgaacgaag tcggcgcctt cttgaagaag gcgcccgagc gcaaggagca gttccacacg     120

atcggggact tcccggtcca gcgcacctac accgctgccg acatcgccga cacgccgctg     180

gaggacatcg gtcttccggg gcgctacccg ttcacgcgcg ggccctaccc gacgatgtac     240

cgcagccgca cctggacgat gcgccagatc gccggcttcg gcaccggcga ggacaccaac     300

aagcgcttca agtatctgat cgcgcagggc cagaccggca tctccaccga cttcgacatg     360

cccacgctga tgggctacga ctccgaccac ccgatgagcg acggcgaggt cggccgcgag     420

ggcgtggcga tcgacacgct ggccgacatg gaggcgctgc tggccgacat cgacctcgag     480

aagatctcgg tctcgttcac gatcaacccg agcgcctgga tcctgctcgc gatgtacgtg     540

gcgctcggcg agaagcgcgg ctacgacctg aacaagctgt cgggcacggt gcaggccgac     600

atcctgaagg agtacatggc gcagaaggag tacatctacc cgatcgcgcc gtcggtgcgc     660

atcgtgcgcg acatcatcac ctacagcgcg aagaacctga agcgctacaa cccgatcaac     720

atctcgggct accacatcag cgaggccggc tcctcgccgc tccaggaggc ggccttcacg     780

ctggccaacc tgatcaccta cgtgaacgag gtgacgaaga ccggtatgca cgtcgacgaa     840

ttcgcgccgc ggttggcctt cttcttcgtg tcgcaaggtg acttcttcga ggaggtcgcg     900

aagttccgcg ccctgcgccg ctgctacgcg aagatcatga aggagcgctt cggtgcaaga     960

aatccggaat cgatgcggtt gcgcttccac tgtcagaccg cggcggcgac gctgaccaag    1020

ccgcagtaca tggtcaacgt cgtgcgtacg tcgctgcagg cgctgtcggc cgtgctcggc    1080

ggcgcgcagt cgctgcacac caacggctac gacgaagcct tcgcgatccc gaccgaggat    1140

gcgatgaaga tggcgctgcg cacgcagcag atcattgccg aggagagtgg tgtcgccgac    1200

gtgatcgacc cgctgggtgg cagctactac gtcgaggcgc tgaccaccga gtacgagaag    1260

aagatcttcg agatcctcga ggaagtcgag aagcgcggtg gcaccatcaa gctgatcgag    1320
```

caggggctggt tccagaagca gattgcggac ttcgcttacg agaccgcgct gcgcaagcag 1380

tccggccaga agccggtgat cggggtgaac cgcttcgtcg agaacgaaga ggacgtcaag 1440

atcgagatcc acccgtacga caacacgacg gccgaacgcc agatttcccg cacgcgccgc 1500

gttcgcgccg agcgcgacga ggccaaggtg caagcgatgc tcgaccaact ggtggctgtc 1560

gccaaggacg agtcccagaa cctgatgccg ctgaccatcg aactggtgaa ggccggcgca 1620

acgatggggg acatcgtcga gaagctgaag gggatctggg gtacctaccg cgagacgccg 1680

gtcttctga 1689

<210> 2
<211> 411
<212> DNA
<213> artificial

<220>
<223> 2-hydroxyisobutyryl-coenzyme A mutase subunit 2

<400> 2
atggaccaaa tcccgatccg cgttcttctc gccaaagtcg gcctcgacgg ccatgaccga 60

ggcgtcaagg tcgtcgctcg cgcgctgcgc gacgccggca tggacgtcat ctactccggc 120

cttcatcgca cgcccgaaga ggtggtcaac accgccatcc aggaagacgt ggacgtgctg 180

ggtgtaagcc tcctgtccgg cgtgcagctc acggtcttcc ccaagatctt caagctcctg 240

gacgagagag gcgctggcga cttgatcgtg atcgccggtg gcgtgatgcc ggacgaggac 300

gccgcggcca tccgcaagct cggcgtgcgc gaggtgctcc tgcaggacac gcccccgcag 360

gccatcatcg actcgatccg ctccttggtc gccgcgcgcg gcgcccgctg a 411

<210> 3
<211> 984
<212> DNA
<213> artificial

<220>
<223> MeaB

<400> 3
atgacttacg ttccctcatc cgccttgctc gagcaactcc gagccggcaa tacctgggcg 60

cttggccgcc tgatctcgcg cgccgaggcc ggtgtggccg aggcgcggcc agcattggcc 120

gaggtctatc ggcacgccgg ctcggcgcat gtgatcggtc tcaccggggt gccggggagt 180

ggcaagtcga ctctcgtggc gaagctcacg gccgccctgc gcaagcgtgg tgaaaaggtc 240

ggcatcgtcg caatcgatcc gtcgagcccg tactcgggcg gtgcgatcct cggcgaccgt 300

atccgaatga ccgaactcgc caacgattcc ggcgtattca tccgcagcat ggccacgcgc 360

ggcgcgacgg ggggcatggc gcgtgccgcc ctcgacgccg tggacctgct ggatgtcgcc 420

ggctatcaca ccatcatcct ggagactgtc ggagtcggtc aggacgaggt ggaggtggcg 480

cacgcatcgg acacgacagt cgtcgtatcg gcgccaggcc ttggagacga gatccaggcc 540

```
atcaaagccg gcgtcctgga aatcgccgac atccatgttg tcagcaagtg tgaccgcgac      600
gacgcgaatc gcacgctcac cgatctcaag cagatgctga cgctcggcac catggtcggg      660
cccaagcgcg catgggcgat cccggtcgtc ggtgtcagtt cgtacacagg cgaaggcgtc      720
gacgacctgc tcggtcgcat cgccgcccac cgccaggcga cggccgacac cgaactcggc      780
cgcgaacggc gccgtcgcgt agccgaattc cgccttcaga agaccgccga gacgctgctc      840
ctggagcgat tcaccaccgg agcgcagccc ttctcgcctg cgctcgcaga cagcctcagc      900
aaccgtgcgt cggatcccta cgccgcagca cgcgaactca tcgcccgaac gatccgcaag      960
gagtactcga atgacctggc ttga                                             984
```